# EUROPEAN PATENT SPECIFICATION

(11) **EP 1 273 355 B1**
(45) Date of publication and mention of the grant of the patent: **31.03.2010**
(21) Application number: 02004089.5
(22) Date of filing: 25.02.2002
(51) Int. Cl.: B05B 17/06, B05B 1/02

(54) **Method of manufacturing a liquid droplet spray device and such spray device**
Verfahren zur Herstellung eines Tröpfchen-Verneblers und ein solcher Vernebler
Procédé de fabrication d'un nébuliseur de goutelettes et un tel nébuliseur

(30) Priority: 23.02.2001 EP 01103653
(43) Date of publication of application: 08.01.2003
(73) Proprietor: Microflow Engineering SA, 2007 Neuchâtel (CH)
(72) Inventor: Hess, Joseph, 2022 Bevaix (CH); Flick, Jean-Marc, 2065 Savagnier (CH); Hu, Bo, 2034 Peseux (CH); Luginbuhl, Philippe, 2518 Nods (CH); Weber, Raphael, 2300 La Chaux-de-Fonds (CH)
(74) Representative: Balsters, Robert

(56) References cited:
- EP-A- 0 985 534
- EP-A- 1 005 917
- EP-A- 1 046 917
- WO-A-00/06388
- US-A- 5 635 337
- US-A- 6 142 607

## Description

The present invention relates to a method of manufacturing a liquid droplet spray device suitable for atomising a liquid substance such as a drug, a fragrance or other aerosolised liquids, as well as the device thus obtained. Such a device may be used, e.g., for perfume dispensers, for inkjet printer heads. for deposition of an array or arrays of droplets on a surface, for fuel injection devices of an engine or for administrating a nebulized drug to a patient by means of his or her respiratory system. Such an administration device, in its simplest form, is commonly called an inhaler. It may be used, e.g., for the controlled administration of drugs or for a variety of therapies using nebulized drug administration including anaesthetics or during minimally invasive surgery. The device delivers the drug, which is in the form of a liquid substance, as a dispersion of atomised droplets. More specifically, the present invention concerns an improved liquid droplet spray device which efficiently creates and which fully expels a liquid droplet spray, as well as a method of manufacturing such.

Various devices are known for atomising a liquid. Document EP 0 516 565 describes an ultrasonic wave nebuliser which atomises water. This apparatus is used as a room humidifier. Vibration is transmitted through the water to the water surface from which the spray is produced. A perforate membrane is provided to retain the water in absence of oscillation.

Typically, inhaler devices use the same principle to atomise the liquid into droplets, see for example the document WO 95/15822.

Furthermore, the droplet size, which depends on the size of the outlet orifices of the perforate membrane, also depends on the vibration frequency. In order to obtain a small droplet, a very high frequency should be used, typically over 1 MHz for droplets of about 10 µm in diameter. This leads to increased power consumption due to the high frequency so that such a device is not suitable for a small battery operated device.

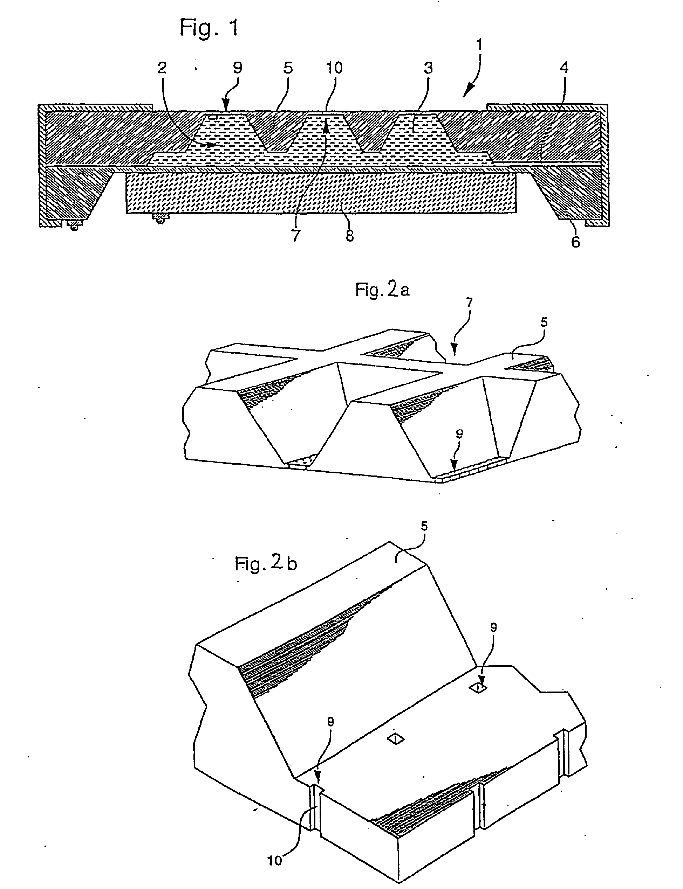

Another liquid droplet spray device is known from the document EP-A-0 923 957 and the document EP-A-1 005 916, both in the name of the present Applicant. A brief description of the liquid droplet spray device known from these documentsis given here while referring to Figure 1.

In this particular embodiment spray device 1 consists of a housing formed of a superposition of a first, or a top substrate 5 and a second, or a bottom substrate 6 in-between which a chamber or a space 2 is formed for containing a liquid substance 3 and thus providing a compression chamber. Top substrate 5 contains outlet means consisting of cavity or cavities 7 which can partly constitute space 2, outlet nozzles 9 and output channels 10 connecting these nozzles to space 2.

Liquid substance 3 enters spray device 1 by way of, e.g., a very low pressure, e.g., around a few millibar, or capillary action. This can be achieved for example by way of at least one input tube or needle 4 through which the liquid substance may be supplied from an external reservoir (not shown) into spray device 1. Spray device 1 further comprises a vibrating element 8, e.g. a piezoelectric element to cause vibration of liquid substance 3 in space 2.

The method of manufacturing this device is carried out by using technology known from the field of semiconductors. Thus, top and bottom substrates may be manufactured in a similar manner e.g. by etching a silicon wafer in a suitable manner, e.g. by wet or dry etching and by using one or more masks or by micro-machining Pyrex wafers. The substrates 5 and 6 are attached to each other, preferably by appropriate bonding technique, such as anodic bonding, so as to form and enclose space 2.

The method of manufacturing the output channel preferably comprises micro-machining the channel by using a deep reactive vertical plasma etching of silicon, e.g. at room temperature or low temperature, and an advanced silicon etch process so as to obtain highly-toleranced straight side-walls of the channel. These prior art documents further describe techniques allowing for such output channels with a straight, non-tapered profile. This provides for a precisely defined pressure drop, droplet size and flow behaviour across output channel 10 for aqueous solutions and suspensions whereas the relatively smooth surface is suited for medications carrying small solid particles, e.g. from less than 1 to approx 2 µm, in suspensions. The same effect can be obtained proportionally with larger dimensions, e.g. with nozzles of 10 µm or larger for example for perfume dispensing applications or in a practical variation of the cited prior art of the applicant by simply using the vertical plasma etching micro-machining method to produce an output channel whose cross-section is divided into two or more identical sub-channels to allow for an even finer control of pressure drop, droplet size and flow behaviour across said channel 10. The cross section of the vertical channel or channel section can be of a suitable geometrical form, e.g. circular, triangular or a suitable geometrical shape such as a cross when the channel consists of several identical sub-channels. The cross section of the cavities 7 can also be of suitable geometrical form or combination of forms.

Figure 2a shows a schematic detailed view of the first, or top substrate of this prior art liquid droplet spray device. The top substrate is shown upside down with respect to Figure 1 in a further practical variation of the cited prior art which has already been shown with an inversion of the bottom substrate, thus further reducing dead space. As can be seen, top substrate 5 comprises the cavities 7, output channels 10 and outlet nozzles 9. The top surface of the substrate-delimiting cavity 7 forms a membrane section in substrate 5. The surface of this membrane section is much larger than the actual nozzle surface, so that it is possible to provide several outlet nozzles 9 on the membrane surface in order to eject more droplets simultaneously. As already mentioned in the cited prior art, it is obvious that cavities 7 are not necessarily tapered but can be straight according to the manufacturing process chosen. Figure 2b shows a close-up view of a part of figure 2a in which it can be seen that the output channels 10 and outlet nozzles 9 may be readily placed according to the specific conditions.

The diameter of a droplet depends on the nozzle hole size "d" for a given frequency of the vibration of the liquid substance and the inlet pressure. In this prior art device where a frequency of around 243 kHz is used, the mean droplet diameter has been found to be around 5 µm, the diameter of the hole of nozzle 9 is around 7 µm and the inlet pressure is a few millibars. One such a droplet thus contains a quantity of around 67 femtolitres (10⁻¹⁵ 1) so that as such the number of nozzles may be determined as a function of the amount to be ejected.

Indeed, the fabrication tolerance Δd of the outlet nozzles is an essential factor in controlling and determining the amount, i.e. the volume "V" of an expelled droplet. In fact, this volume V depends on d³ (V= 1/6 * Πd³), d being the diameter of the outlet nozzle. For example, if d = 5 µm, and Δd = ±0.5 µm, the droplet volume V may vary from 47.5 (d= 4.5) to 87 (d=5.5) which is a variation of 83%.

Furthermore, it is known that the pressure drop across the output channel depends on d⁴, so it may be understood that the outlet diameter, the channel diameter, its cross-section, as well as any combination of varying micro-machined cross-sections of the outlet channel and nozzle are an important factor in the structure of the liquid droplet spray device. In fact, this influence of the channel diameter size can be used to determine and vary the pressure drop and the velocity of the nebulized spray.

It is also known that the droplet diameter varies with certain physico-chemical properties of the liquid such as surface tension and viscosity. It is therefore important as shown in the cited prior art to be able to adapt the physical and electrical device parameters (frequency and amplitude) according to the liquid to be expelled and the desired droplet characteristics.

The applicant has now found that under certain circumstances the droplet size, the velocity and flow rate also vary with the voltage applied to the vibrating element. These conditions arise when the pressure drop across the outlet channel is low enough to allow for, for a given range, for example a mean diameter between 5 and 10 µm, the modulation of the droplet size, velocity and flow rate by the energy input alone, hence by the supply voltage.

Consequently, it seems possible to vary the geometric parameters of the outlet means in order to reach a certain droplet diameter, and then vary that size of the ejected droplets by varying the applied voltage.

It is however necessary to apply an innovative micro-machining technique to create a device that reaches such behaviour. Whilst semiconductor fabrication methods such as disclosed by applicant in documents EP-A-0 923 957 and EP-A-1 005 916, cited above, allow to etch the desired straight channels, it is difficult to further improve on the control of the pressure drop while at the same time retaining the control over the droplet size.

It is further known to create nozzle bodies having step-shaped channels. For example, the document EP-A-0 985 534 describes a method producing an inkjet printer head in which step-shaped nozzle channels are formed by dry etching techniques using induction coupled plasma (ICP) discharge. The nozzle formed has a cross-section made smaller stepwise toward the front end thereof. First of all, a resist film is formed on a surface of a silicon monocrystalline substrate. A first opening pattern is formed by half-etching the resist film at a portion corresponding to the rear, wider end of the nozzle channel. Next, a second opening pattern smaller than the first pattern is formed by full-etching the half-etched resist at a portion corresponding to the front, narrower end of the nozzle channel. Then, the nozzle,channel is formed by applying dry etching by plasma discharge to the exposed second, narrower portion of the surface of the silicon monocrystalline substrate. After this, a second dry etching is carried out by full-etching the remaining hall-etched portion and by continuing the plasma etching of both the narrow and the wide portions until a through hole is obtained.

However, in this technique using plasma etching, notching inevitably occurs at the output ends of the nozzle channel. In fact, when etching the wider portion, the etching will continue in the narrower portion too due to etched ions located in this narrower portion of the nozzle channel. When reaching the end of the channel, a through hole is created, but the remaining ions will also etch away the edges of the through hole of the channel. Naturally, notched output nozzles complicate the droplet formation. In fact they favour the spreading of liquid in close vicinity of the nozzles and thus generate larger droplets than intended or prevent droplet formation all together by this wetting of the active surface.

It is, therefore, an object of the present invention to provide a method of manufacturing a liquid droplet spray device as well as the thus obtained device which overcomes the above-mentioned inconveniences and which allows for a controllable and programmable droplet size and flow rate.

It is another object of the present invention to provide such a device that is simple, reliable to manufacture, small in size and low in energy consumption and cost.

Thus, the present invention concerns a method of manufacturing a liquid droplet spray device and also a liquid droplet spray device as defined in the appended claims.

The present invention also concerns a method of manufacturing a nozzle body for such a liquid droplet spray device, as also defined in the claims.

Thanks to the specific shape and arrangement of the outlet means of the spray device according to the present invention a programmable droplet size, velocity and flow rate of the expelled droplet may be obtained in a relatively simple manner. This can provide for an economic advantage, as the same device platform can be used to provide a different droplet size distribution, flow rate or exit velocity by simply changing the supply voltage applied to the piezoelectric vibrating element.

Other features and advantages of the liquid spray device according to the present invention will become clear from reading the following description, which is given solely by way of a non-limitative example thereby referring to the attached drawings in which:
- FIGURE 1 is a schematic cross-section of a prior art liquid droplet spray device,
- FIGURE 2a to 2b show schematic detailed views of the top substrate of the prior art liquid droplet spray device of Figure 1,
- FIGURE 3a to 3c show schematic cross-sections of a preferred embodiment of the liquid droplet spray device according to the present invention, and
- FIGURE 4 shows the steps of a method of fabricating, via an inventive differential plasma etch, a possible embodiment of a liquid droplet spray device according to the present invention.
- FIGURE 5 shows a bottom view of the first substrate incorporating a compression chamber and passive valves in a single space, and
- FIGURE 6 shows steps of another method of manufacturing the present liquid droplet spray device.

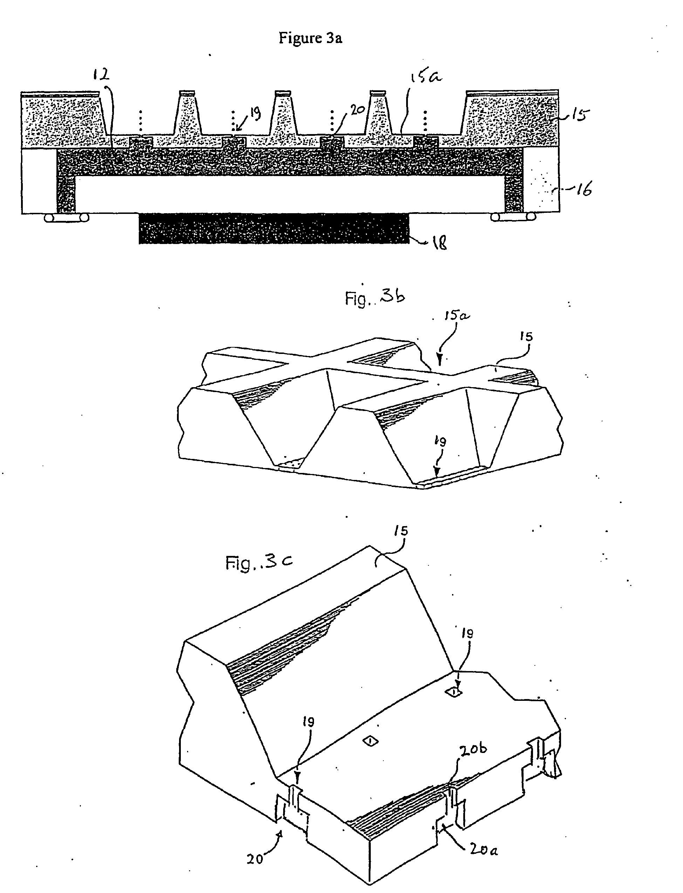

An example of a preferred embodiment will be described hereafter. The present invention thus concerns a method of manufacturing a liquid droplet spray device for nebulizing a liquid substance. The present invention also concerns the thus obtained spray device. For ease of understanding, the structure of the liquid droplet spray device itself will first be described while referring to figures 3a to 3c. In principle, the spray device may be rather similar to the above described prior art spray device of the present applicant, except for the outlet means.

Thus, the present spray device also comprises a housing or a substrate. This housing may be formed of a first substrate 15 and a second substrate 16, in a rather similar manner as shown in Figure 1. However, as shown in figure 3, first substrate 15 is placed upside down compared to first substrate 5 of figure 1. The housing may, however, only consist of a single substrate as will be explained in detail later on. Substrate 15 is provided with membrane sections 15a which are thinner sections of the substrate obtained by etching away parts of the substrate using appropriate methods of etch stop to guarantee homogeneous membrane thickness. The manner of obtaining such membrane sections is similar to that as described in the above referenced prior art document EP-A-0 923 957, and is well known to the skilled person from the field of semiconductor etching.

The etching may be done by wet or dry etching resulting in inclined or in straight sidewalls of the substrate portion leading away from the membrane section.

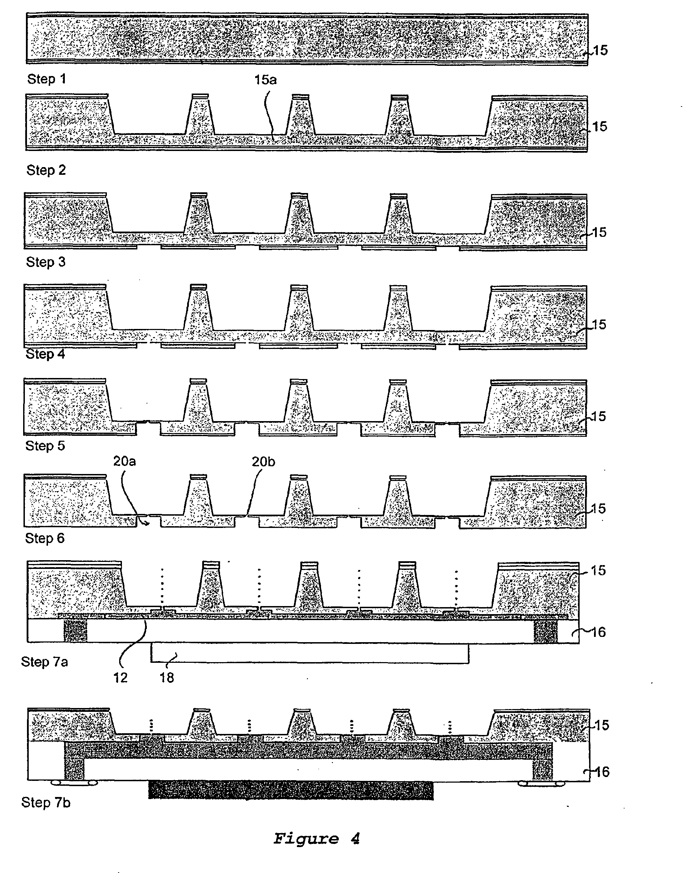

As can be seen in Figure 3, a space 12 for containing the liquid substance is provided within the housing, between the two substrates 15 and 16. Such space can be created by etching a recess in the bottom surface of first substrate 15, the bottom substrate 16 presenting a flat surface towards the inside of space 12 such as shown in FIGURES 3 and 4. Such space can also be created in the bottom substrate 16 by wet or dry etch as will be described further on. The second substrate can also be advantageously replaced directly by the vibrating element 18 which for the purpose of protection has been suitably passivated as described in EP-A-0 923 957.

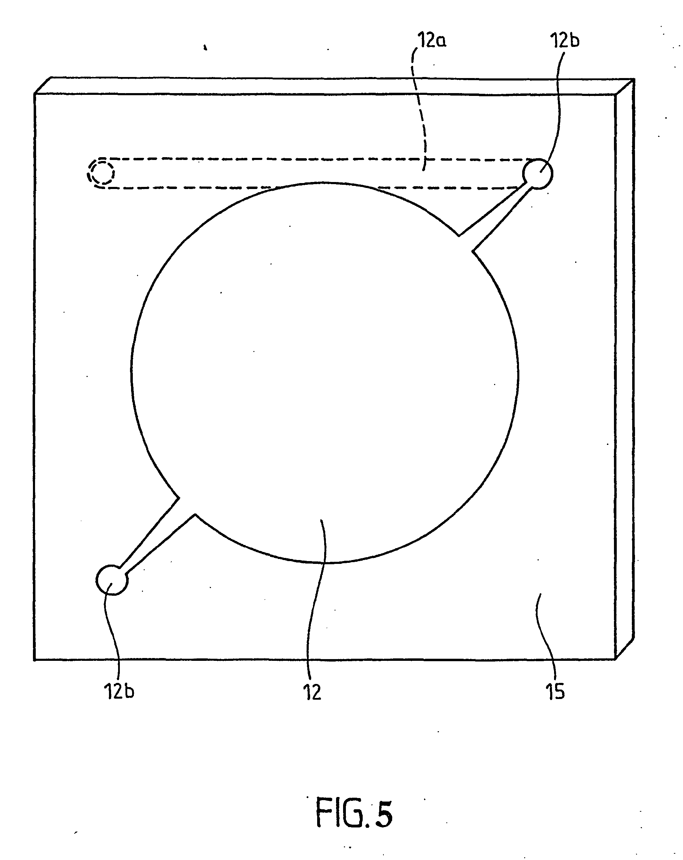

As shown in Figure 5, space 12 is preferably of a round cross-section in order to facilitate filling and air evacuation and may incorporate a geometry for creating passive valves at its inlet and outlet. It may further be dimensioned or complemented, e.g. by connecting to an internal or external dosage buffer volume 12a, to contain a required dosage amount such that principal volume 12 together with buffer volume 12a correspond to a desired unit dose which may be larger than the internal volume of space 12. Thus, the desired unit dose may have a maximum dosage corresponding to the total volume of space 12, i.e. of principal volume 12 plus buffer volume 12a. In such a way space 12 is at first, e.g. before starting the droplet size generation, completely filled with the liquid to be expelled and remains filled for most of the operation when liquid is expelled roughly as fast as it is aspirated from buffer volume 12a, resulting in a stable operating condition for most of the operation, especially if the content of buffer volume 12a is larger than that of space 12. This buffer volume 12a allows for the spray device to be filled with a different dose for each intended use, ranging from the approximate volume of space 12 to the total volume of space 12 plus buffer volume 12a. Buffer volume 12a is realised preferably as a capillary meander or other geometrical configuration having a cross-section that facilitates capillary action thus providing easier priming of space 12 and buffer 12a. Further, appropriate means, such as a capillary channel 12b for supplying the liquid substance to and allowing exiting from space 12 is provided as known from the mentioned prior art. Such capillary channel can be advantageously configured to act as a passive valve, which is known as such, to allow for the liquid substance to enter and exit the space.

These features are highly advantageous when delivering for example insulin doses with a varying dosage volume depending on the glucometer reading at the time of use. It may of course also be advantageous in other applications where a variable dosage is indicated.

At least one outlet nozzle 19 and at least one output channel 20 for connecting space 12 to each outlet nozzle 19 are further provided in the thinner membrane section 15a of substrate 15. It is of course important that the output channel 20 has straight sidewalls so as to be able to define the pressure drop across the channel when a droplet is ejected, as already explained in detail in the above-mentioned prior art EP-A-0 923 957.

A vibrating element such as a piezoelectric element 18 is disposed on the housing to vibrate the liquid substance in space 12. Preferably, the vibrating element is arranged directly on the first substrate 15 or on a thinner section of the second substrate 16, e.g. acting as a membrane for transmitting a certain compression as well as the vibration to the liquid contained in space 12 and vibration to the total structure such as described in the above-mentioned prior art. When the liquid is excited at an appropriate frequency and under the appropriate pressure, it will be ejected as a spray of droplets through the outlet nozzles with a very low exit velocity. The preferred operation is at the fundamental resonance frequency of vibrating element 18 or at subsequent harmonics.

The transition of output channel 20 from space 12 to outlet nozzle 19 is not only straight, but is also step-shaped. As can be seen in figure 3c, output channel 20 consists of a lower part 20a and an upper part 20b. Lower part 20a of output channel 20 has a larger diameter than upper part 20b and can have the same or a different length as the upper part. Lower part 20a is arranged adjacent space 12 containing the liquid substance which is to be expelled.

Thanks to the stepped shape of the output channel 20, the excited liquid is forced at a higher pressure into the upper part 20b of the output channel. Thus, the eventual size of the droplet results mainly from the liquid volume that is contained in the smaller upper part 20b. Furthermore, the outlet nozzle is also straight without any notching at its periphery, thanks to the inventive method of manufacturing the spray device, as will be explained in detail further on.

As explained above, for a given liquid the diameter and the volume of the expelled droplet depend mainly on the pressure drop across the output channel and also on the applied voltage, amplitude and frequency.

Thus, as of a certain level of energy balance, the Applicant has found it to be possible, and practical, with the present structure to vary the droplet size by only varying the voltage applied to the vibrating element 18.

Further, thanks to the stepped shape of the output channel and the straight outlet nozzle, when the same power is used to create the droplet spray, a smaller droplet can be generated as compared to the mentioned prior art devices.

Experiments have shown that, for a given outlet channel diameter, if the applied voltage increases, the mean droplet size decreases. Indeed, the following results were obtained:

| *Applied voltage (V)* | *Mean droplet size (µm)* |
|---|---|
| 5 | > 18 |
| 10 | 10 - 12 |
| 20 | 7 |
| 30 | 3-4 |

Applicant has found that at virtually no input pressure, at 30 V and approximately 250 kHz applied to the vibrating element, more than 80% of the droplets were smaller than the diameter of the upper part of channel 20b.

It may thus be understood that it is possible to vary the droplet size, but with it the flow rate and also the exit velocity by varying the applied voltage so that a programmable platform for a liquid droplet spray device may be obtained. Consequently, it is possible to provide largely identical liquid droplet spray devices for different applications by simply varying the applied voltage. For example, the same spray device may be used as an inhaler for systemic, deep lung applications which require droplets of less than 3,3 µm or for upper lung treatment which requires droplets of less than 5,6 µm or for various types of liquids which require a different energy input to obtain the same droplet size.

According to the present invention, such a liquid droplet spray device is manufactured by using the following method which is explained whilst referring to FIGURES 4 and 5. In principle, it is possible to etch two silicon wafers, one corresponding to a plurality of first substrates 15 and the other corresponding to a plurality of bottom substrates 16. However, it may be appreciated that the structure of the present liquid droplet spray device may also be realised by sandwiching different materials such as Si, SiO₂, SiN, SU-8, metal, and the like, in suitable combinations, or by successive depositions of such materials. A detailed explanation of the manufacturing of the first substrate 15 will be provided here. Indeed, this substrate has a more complicated shape than the bottom substrate.

As shown in step 1 of FIGURE 4, first an oxide mask is deposited on both surfaces of a substrate which will form the first substrate 15.

Next, in step 2, the top surface is etched using for example a wet etching process, advantageously with an etch stopper (not shown) so as to obtain the thinner membrane sections 15a.

In step 3, two possibilities exist. In one case the oxide at the bottom surface of first substrate 15 is opened, defining large openings corresponding to the lower, wider, parts 20a of output channels 20, in this case space 12 is machined into substrate 16. In another case, the oxide is etched partially in order to shape space 12 into substrate 15 followed by an additional etch which defines the large openings corresponding to the lower, wider, parts 20a of output channels 20 (not fully shown in Fig. 4).

In step 4, after a photo-resist has been applied defining the small openings corresponding to upper parts 20b of output channels 20, a plasma etching, preferably by using an ICP (Inductively Controlled Plasma) technique, of the silicon is carried out to obtain the shape of the small openings and is stopped at a suitable depth. After stripping of the resist followed by a differential plasma etching, the stepped configuration of output channels 20 with the desired proportions of the parts 20a and 20b is obtained without however opening the top of upper channel part 20b.

Indeed, according to the present invention, this differential plasma-etching step is stopped before the etching pierces, i.e. traverses the substrate. In fact, the present inventors have found that when piercing the substrate during such plasma-etching step, notching occurs resulting in slightly tapered outlet nozzles which could have a negative influence on the control of the droplet size and on its directivity when ejected from the nozzle.

In fact, a third plasma etch step is carried out either to create the space 12 locally into substrate 15 and to open upper channel part 20b or the third plasma etch step is carried out over the entire wafer surface in order to open upper channel part 20b in case that space 12 is to be machined into substrate 16, as shown respectively in steps 7b or 7a of Fig 4.

In both cases the surface to be etched is very large which results in a lower etching speed of the channel, i.e. the upper, narrow part 20b will be etched relatively slowly. Thus. the actual piercing will then occur at a very low speed which will avoid any notching effects.

Thus, according to the present invention, it is possible to avoid notching and thereby obtain straight through holes, i.e. straight step-shaped output channel by a correct choice of the surfaces to be differentially etched. In fact, the ratio of the large etching surface (corresponding to space 12) to the small etching surface (corresponding to the upper, narrow channel part 20b) should be chosen such that the etching speed will be low enough in the small etching surface that notching is avoided.

After that last differential plasma etch cycle the wafer is turned and the top surface of the first substrate 15 is etched with the same plasma etcher, preferably in only one step which is stopped in the passivation mode in order to provide a hydrophobic quality of that top surface.

This innovative etching process is called differential etching because the larger exposed surface will be etched faster than the smaller exposed surface, i.e. the "bottom" of the narrow channel part. During this etching step, the narrower channel will be etched to become slightly longer so as to eventually correspond to the actual desired dimensions of upper part 20b of output channel 20, but this narrower part will also be "pushed forward" with respect to the larger opened portion of the silicon by the etching so as to eventually pierce the substrate with the third plasma-etching step.

A skilled person will readily realise that the thinner section for accommodating the piezoelectric element may be obtained by machining it into silicon or Pyrex wafers. This second substrate may also be directly replaced by a suitably passivated vibrating element 18. Any further shape changes may also be conceived by applying the usual techniques in the field.

Finally, in a further step, indicated as step 7a and 7b in figure 4, the bottom surface of first substrate 15 and the top surface of second substrate 16 are bonded together, preferably by using anodic bonding so as to form the housing of the liquid droplet spray device thereby enclosing space 12.

As mentioned above, it is possible to apply a further differential etching step to first substrate 15 to etch away a part of first substrate 15 so as to obtain space 12 directly in first substrate 15. It is further also possible to etch, either at the same time or in a separate step, the buffer volume 12a and/or passive valves 12b. Then, second substrate 16 may be applied to first substrate 15 so as to close space 12. However, as also mentioned briefly above, instead of using the second substrate, it is also possible to directly bond the vibrating element 18, whose surface is preferably suitably treated and protected beforehand, to first substrate 15 so as to enclose space 15.

A selective hydrophilic coating, such as an amorphous material such as SiO2, may further be applied to provide a protective layer around the inside surface of space 12 and/or of the output channels 20 to avoid any contamination of the liquid substance by the material of these surfaces and to improve wettability in certain parts. This hydrophilic coating which may be applied as a selective, patterned coating and is advantageously coupled with a selective, patterned hydrophobic coating in certain areas of space 12 and on the outside of first substrate 15.

Alternatively to applying a plasma etch, using the same production equipment as for the innovative differential plasma etching process described earlier, on the top surface of substrate 15 and stopping it in the passivation mode, a hard amorphous carbon film may be provided on first substrate 15 in order to maintain the protective aspect of its surfaces and at the same time to reduce the internal and external stiction due to capillary forces in space 12, and especially on the outside of substrate 15. This hard amorphous carbon film, e.g. diamond-like carbon (DLC) or fluorinated DLC (F*DLC) is provided, preferably in a selective patterned manner in these areas. Other hydrophobic coatings such as nitride or Teflon might be deposited by spinning and curing, by plasma or by other suitable methods. Such selective film coating also allows for a more complete emptying of space 12 and avoids stiction of liquid on the outside surface of substrate 15 due to low surface energy.

The present Applicant has found that such surface property specific coating influences and improves droplet size dispersion and provides an even better monodispersive pattern released by the spray device. These coatings may be carried out as described in the document US-A-5 462 839.

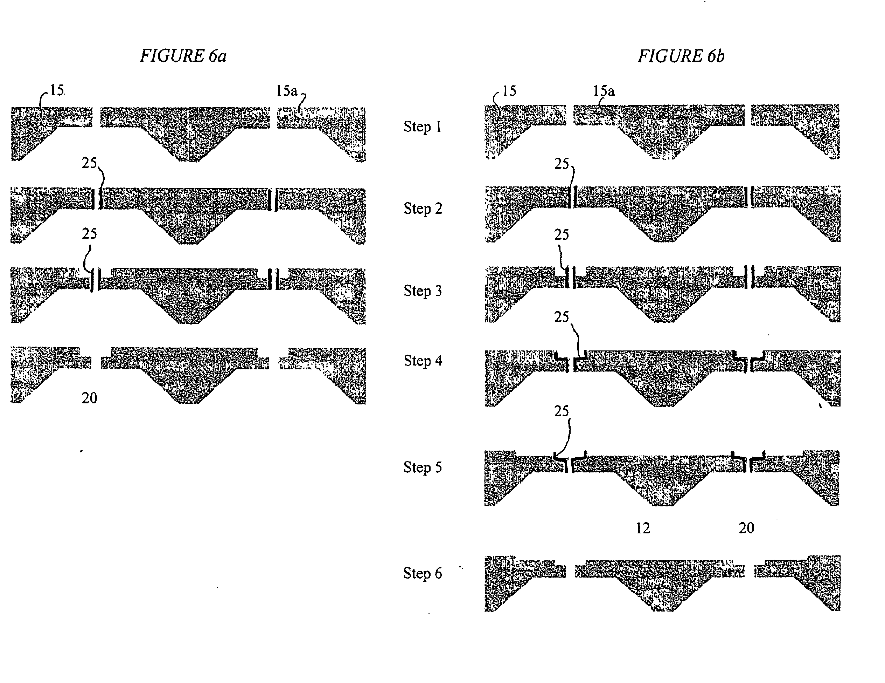

Another method of manufacturing the present liquid droplet spray device, and/or the nozzle body thereof, will now be described while referring to Figures 6a and 6b. Only steps that are different from those described above will be explained in detail. In fact, following steps 1 and 2 shown in Figure 4, so as to obtain the thinner membrane sections 15a of first substrate 15, it is possible to directly etch, in any conventional manner, the bottom surface of a membrane section to obtain a channel, the diameter of which corresponds substantially to the narrow upper portion 20b of the output channel 20. Next, the whole wafer surface is etched as described earlier in order to pierce the substrate without notching at the channel outlet nozzle 19. Thus, a channel traversing the membrane section 15a is obtained, as shown in step 1 of both figures 6a and 6b.

Following this, the inner sidewalls of the channel are covered with a protective layer 25 such as an oxide, as shown in step 2 of Figures 6a and 6b. Then, a resist film is formed on the bottom surface of first substrate 15 and an opening is defined corresponding to the lower wider portion 20a of output channel 20 (not shown). Then, the lower portion 20a is formed by etching first substrate 15 so as to obtain the lower portion 20a of the output channel, as shown in step 3 of figures 6a and 6b.

At this point, similar to what has been described above, there are 2 possibilities depending on into which substrate space 12 is to be integrated.

In a first possibility the oxide in the narrow upper portion 20b is removed, as shown in step 4 of figure 6a, and first substrate 15 is substantially ready to be bonded with second substrate 16 in which space 12 has been machined as described earlier.

In the other version, the sidewalls of the lower portion 20 a are also covered with a protective layer same as the oxide protecting the inner sidewalls of the upper portion 20b, as shown in step 4 of figure 6b. Then, another resist is applied on the bottom surface of first substrate 15 and an opening is defined corresponding to the recess which is to constitute space 12 (not shown). After this, the bottom surface of first substrate 15 is again etched so as to obtain the recess, forming space 12, as shown in step 5 of figure 6b. Then the oxide is removed as shown in step 6 of figure 6b.

In this manner, it is thus also possible to obtain the same structure of the liquid droplet spray device according to the present invention having a step-shaped output channel with a recess whereby the outlet nozzle at the end of the output channel does not show notching influences when the substrate is pierced. Although in this alternative second method, more steps are required than in the above-described first method, the steps used here can be conventional steps thus allowing for a reliable manufacturing of the liquid droplet spray device.

Having described a preferred embodiment of this invention, it will now be apparent to one of skill in the art that other embodiments incorporating its concept may be used. It is felt, therefore, that this invention should not be limited to the disclosed embodiment, but rather should be limited only by the scope of the appended claims.

For example, the same liquid droplet spray device may not only be used for an inhaler in respiratory therapies, but it may generally be used for creating nebulized liquids of different physico-chemical compositions, e.g. using aqueous or alcoholic or other liquid substances.

## Claims

1. Liquid droplet spray device for atomising a liquid substance, comprising:
- a housing consisting at least of a first substrate (15) having at least one membrane section (15a) that is thinner than the rest of said first substrate (15),
- a space (12) within the housing for containing the liquid substance,
- means for supplying said liquid substance to said space (12),
- an outlet means arranged in said membrane section (15a) and comprising at least one outlet nozzle (19) arranged in a first main surface of said first substrate (15) and at least one output channel (20) connecting said space (12) to each of said at least one outlet nozzle (19), and
- a vibrating element (18) disposed to vibrate liquid in said space (12) so as to eject said liquid substance as a spray through said outlet nozzles (19),
**characterised in that** each output channel (20) has a stepped shape having a lower portion (20a) and an upper portion (20b), said lower portion being arranged adjacent said space (12) and being of larger diameter than said upper portion (20b), each output channel portion (20a, 20b) having straight side walls, and **in that**
said rest of said first substrate (15) consists of sections that are thicker than said membrane section (15a) and that are located on the side of the first main surface of said first substrate (15).

2. Liquid droplet spray device according to claim 1, further **characterised in that** said rest of said first substrate (15) consists of thicker sections having sidewalls leading away from said membrane sections (15a).

3. Liquid droplet spray device according to claim 1 or 2, further **characterised in that** said first substrate (15) has a recess in its other main surface, which constitutes said space (12).

4. Liquid droplet spray device according to claim 1, further **characterised in that** said vibrating element (18) is round and of a smaller cross-section than said space (12) and is arranged to operate at its fundamental resonance frequency or subsequent harmonics so as to eject said liquid substance as a spray through said outlet nozzles (19),

5. Liquid droplet spray device according to claim 3, wherein said housing further comprises a second substrate (16), and wherein said space (12) constituted by said recess is closed off by said second substrate (16).

6. Liquid droplet spray device according to claim 1 or 2, wherein said housing further comprises a second substrate (16), and wherein said space (12) is provided in said second substrate (16).

7. Liquid droplet spray device according to anyone of the preceding claims, further comprising a passive valve (12b) arranged in physical combination with said space (12) for facilitating the homogeneous operation of said device by providing a homogenous filling of said space (12) with said liquid substance.

8. Liquid droplet spray device according to anyone of the preceding claims, wherein said space consists of a principal volume (12) and a buffer volume (12a), said buffer volume being dimensioned such that the total volume of said principal volume (12) together with said buffer volume (12a) corresponds to a maximum dosage, so that a desired variable dosage depending on the specific usage of said spray device may be contained in said space, said variable dosage being equal to or less than said maximum dosage.

9. Method of manufacturing a liquid droplet spray device for atomising a liquid substance according to claim 6, the method comprising the following steps:
a. depositing an oxide mask on both main surfaces of a silicon substrate which will form the first substrate (15),
b. etching a first main surface of said first substrate (15) so as to obtain the thinner membrane sections (15a),
c. applying a first photo-resist to the other main surface of said first substrate (15) defining large openings corresponding to the lower portion (20a) of said output channels (20) and then etching away the oxide mask to open access to the silicon of said first substrate (15),
d. applying a second photo-resist to define small openings corresponding to the upper portion (20b) of said output channels (20) and then using a partial plasma etching of the silicon to obtain a narrow channel which is narrower and shorter than the actual upper portion (20b) will be, and stripping the second photo-resist,
e. etching the opened silicon by using a differential plasma etching until the substrate (15) is nearly pierced,
f. using a plasma etching on the other main surface of said first substrate (15) to pierce said substrate (15), the ratio of the surface to be etched with respect to the surface corresponding to the nozzle opening of said upper portion (20b) being such that the etching speed of said nozzle is slow enough to avoid notching of the outlet nozzle (19),
g. etching a second silicon substrate so as to form said second substrate (16) with a space (12) therein,
h. bonding together said first substrate (15) and said second substrate (16) to form said housing of said liquid droplet spray device,
i. providing means for supplying said liquid substance to said space (12), and
j. providing said vibrating element (18).

10. Method of manufacturing a liquid droplet spray device for atomising a liquid substance according to claim 3, the method comprising the following steps:
a. depositing an oxide mask on both main surfaces of a silicon substrate which will form the first substrate (15),
b. etching a first main surface of said first substrate (15) so as to obtain the thinner membrane sections (15a),
c. applying a first photo-resist to the other main surface of said first substrate (15) defining large openings corresponding to the lower portion (20a) of said output channels (20) and then etching away the oxide mask to open access to the silicon of said first substrate (15),
d. applying a second photo-resist to define small openings corresponding to the upper portion (20b) of said output channels (20) and then using a partial plasma etching of the silicon to obtain a narrow channel which is narrower and shorter than the actual upper portion (20b) will be, and stripping the second photo-resist,
e. etching the opened silicon by using a differential plasma etching until the substrate (15) is nearly pierced,
f. applying a third photo-resist to define a large opening corresponding to the space (12) and then using a plasma etching to obtain said recess constituting said space (12) and to pierce said substrate (15), the ratio of the surface to be etched corresponding to said space with respect to the surface corresponding to the nozzle opening of said upper portion (20b) being such that the etching speed of said nozzle is slow enough to avoid notching of the outlet nozzle (19),
g. providing means for supplying said liquid substance to said space (12),
h. providing said vibrating element (18), and
i. bonding together said first substrate (15) and said vibrating element (18) to form said housing of said liquid droplet spray device.

11. Method of manufacturing a liquid droplet spray device for atomising a liquid substance according to claim 3, the method comprising the following steps:
a. depositing an oxide mask on both main surfaces of a silicon substrate which will form the first substrate (15),
b. etching a first main surface of said first substrate (15) so as to obtain the thinner membrane sections (15a),
c. applying a first photo-resist to the other main surface of said first substrate (15) defining large openings corresponding to the lower parts (20a) of said output channels (20) and then etching away the oxide mask to open access to the silicon of said first substrate (15),
d. applying a second photo-resist to define small openings corresponding to the upper parts (20b) of said output channels (20) and then using a partial plasma etching of the silicon to obtain a narrow channel which is narrower and shorter than the actual upper part (20b) will be, and stripping the second photo-resist,
e. etching the opened silicon by using a differential plasma etching until the substrate (15) is nearly pierced,
f. applying a third photo-resist to define a large opening corresponding to the space (12) and then using a plasma etching to obtain said recess constituting said space (12) and to pierce said substrate (15), the ratio of the surface to be etched corresponding to said space with respect to the surface corresponding to the nozzle opening of said upper portion (20b) being such that the etching speed of said nozzle is slow enough to avoid notching of the outlet nozzle (19),
g. etching a second silicon substrate so as to form said second substrate (16),
h. bonding together said first substrate (15) and said second substrate (16) to form said housing of said liquid droplet spray device,
i. providing means for supplying said liquid substance to said space (12), and
j. providing said vibrating element (18).

12. Method of manufacturing a liquid droplet spray device according to claim 9, 10 or 11, further comprising, after step "e" and before step "g", a step of applying a selective hydrophilic coating to provide a protective layer around the inside surface of space (12) and/or of the output channels (20) to avoid any contamination of the liquid substance by the material of these surfaces and to improve wettability in certain parts.

13. Method of manufacturing a liquid droplet spray device according to claim 12, further comprising the step of applying a selective hydrophobic coating in those areas of said space (12) which do not contain the hydrophilic coating and on the outer surface of said first substrate (15).

14. Method of manufacturing a liquid droplet spray device according to claim 11, further comprising an additional step of plasma etching the first main surface of said first substrate (15) which is stopped in the passivation mode to provide a hydrophobic surface quality using the same production equipment as for the other differential plasma etch steps.

15. Method of manufacturing a liquid droplet spray device according to claim 13 or 14, further comprising the step of applying a hard amorphous carbon film on said hydrophobic coating.

16. Method of manufacturing a liquid droplet spray device for atomising a liquid substance according to claim 3, the method comprising the following steps:
a. depositing an oxide mask on both main surfaces of a silicon substrate which will form the first substrate (15),
b. etching a first main surface of said first substrate (15) so as to obtain the thinner membrane sections (15a),
c. applying a first photo-resist to the other main surface of said first substrate (15) defining narrow openings corresponding to the upper portion (20b) of said output channels (20) and then etching away the oxide mask to open access to the silicon of said first substrate (15),
d. etching the silicon to obtain a through hole traversing the first substrate (15) such that no notching occurs,
e. applying a protective coating (25) to the inner sidewalls of said through hole,
f. applying a second photo-resist on the other main surface of said first substrate (15) to define large openings corresponding to the lower portion (20a) of said output channels (20) and then etching the silicon to obtain a large channel corresponding to the lower portion (20a), and stripping the second photo-resist,
g. applying a protective coating (25) to the inner sidewalls of the now step-shaped through hole corresponding to the step-shaped output channel (20),
h. applying a third photo-resist on the other main surface of said first substrate (15) to define a large opening corresponding to the space (12) and then etching the silicon to obtain said stepped shaped recess constituting said space (12),
i. removing said protective coating from said inner sidewalls of said through hole,
j. providing means for supplying said liquid substance to said space (12),
k. providing said vibrating element (18), and
l. bonding together said first substrate (15) and said vibrating element (18) to form said housing of said liquid droplet spray device.

17. Method of manufacturing a nozzle body of a liquid droplet spray device, said nozzle body consisting of a substrate having at least one membrane section that is thinner than the rest of the substrate, wherein an outlet means is arranged in said membrane section which comprises at least one outlet nozzle (19) arranged in a first main surface of said first substrate (15) and at least one output step-shaped channel (20) connecting one main surface of the substrate to the other main surface of the substrate, said output channel (20) having straight side-walls and having a lower wide portion (20a) and an upper narrow portion (20b),
the method comprising the following steps:
a. depositing an oxide mask on both surfaces of said substrate which will form the nozzle body,
b. etching a first main surface of said substrate so as to obtain the thinner membrane sections (15a),
c. applying a first photo-resist to the other main surface of said substrate defining large openings corresponding to the lower parts (20b) of said output channels (20) and then etching away the oxide mask to open access to the silicon of said substrate,
d. applying a second photo-resist to define small openings corresponding to the upper parts (20b) of said output channels (20) and then using a partial plasma etching of the silicon to obtain a narrow channel which is narrower and shorter than the actual upper part (20b) will be, and stripping the second photo-resist,
e. etching the opened silicon by using a differential plasma etching until the substrate is nearly pierced,
f. plasma-etching the other main surface of said substrate so as to pierce said substrate (15), the ratio of the surface to be etched of said substrate with respect to the surface corresponding to the nozzle opening of said upper portion (20b) being such that the etching speed of said nozzle is slow enough to avoid notching of the nozzle.

## Patentansprüche

1. Flüssigkeitströpfchen-Versprühvorrichtung zum Versprühen einer flüssigen Substanz, mit:
einem Gehäuse, das zumindest aus einem ersten Träger (15) mit mindestens einem Membranteil (15a) besteht, der dünner als der Rest des ersten Trägers (15) ist;
einem Zwischenraum (12) in dem Gehäuse zum Aufnehmen der flüssigen Substanz;
Mitteln zum Zuführen der flüssigen Substanz zu dem Zwischenraum (12);
Auslassmitteln, die in dem Membranteil (15a) angeordnet sind und mindestens eine Auslassdüse (19), die in einer ersten Hauptfläche des ersten Trägers (15) angeordnet ist, und mindestens einen Auslasskanal (20) aufweisen, der den Zwischenraum (12) mit jeder der mindestens einen Auslassdüse (19) verbindet; und
einem Schwingelement (18), das so eingerichtet ist, dass es Flüssigkeit in dem Zwischenraum (12) in Schwingung versetzt, um die flüssige Substanz als eine Sprühflüssigkeit durch die Auslassdüse (19) auszustoßen,
**dadurch gekennzeichnet, dass**
jeder Auslasskanal (20) eine abgestufte Gestalt mit einem unteren Teil (20a) und einem oberen Teil (20b) hat, wobei der untere Teil angrenzend an den Zwischenraum (12) angeordnet ist und einen größeren Durchmesser als der obere Teil (20b) hat und jeder Auslasskanalteil (20a, 20b) gerade Seitenwände hat, und
der Rest des ersten Trägers (15) aus Teilen besteht, die dicker als der Membranteil (15a) sind und die sich auf der Seite der ersten Hauptfläche des ersten Trägers (15) befinden.

2. Flüssigkeitströpfchen-Versprühvorrichtung nach Anspruch 1, die weiterhin **dadurch gekennzeichnet ist, dass** der Rest des ersten Trägers (15) aus dickeren Teilen besteht, die Seitenwände haben, die von den Membranteilen (15a) weg führen.

3. Flüssigkeitströpfchen-Versprühvorrichtung nach Anspruch 1 oder 2, die weiterhin **dadurch gekennzeichnet ist, dass** der erste Träger (15) in seiner anderen Hauptfläche eine Aussparung hat, die den Zwischenraum (12) bildet.

4. Flüssigkeitströpfchen-Versprühvorrichtung nach Anspruch 1, die weiterhin **dadurch gekennzeichnet ist, dass** das Schwingelement (18) rund ist und einen kleineren Querschnitt als der Zwischenraum (12) hat und so eingerichtet ist, dass es auf seiner Grundresonanzfrequenz oder den nachfolgenden Oberschwingungen arbeitet, um die flüssige Substanz als eine Sprühflüssigkeit durch die Auslassdüse (19) auszustoßen.

5. Flüssigkeitströpfchen-Versprühvorrichtung nach Anspruch 3, **dadurch gekennzeichnet, dass** das Gehäuse weiterhin einen zweiten Träger (16) aufweist und der Zwischenraum (12), der von der Aussparung gebildet wird, von dem zweiten Träger (16) verschlossen wird.

6. Flüssigkeitströpfchen-Versprühvorrichtung nach Anspruch 1 oder 2, **dadurch gekennzeichnet, dass** das Gehäuse weiterhin einen zweiten Träger (16) aufweist und der Zwischenraum (12) in dem zweiten Träger (16) vorgesehen ist.

7. Flüssigkeitströpfchen-Versprühvorrichtung nach einem der vorhergehenden Ansprüche, die weiterhin ein passives Ventil (12b) aufweist, das in physischer Kombination mit dem Zwischenraum (12) so angeordnet ist, dass es den gleichmäßigen Betrieb der Vorrichtung dadurch ermöglicht, dass es den Zwischenraum (12) gleichmäßig mit der flüssigen Substanz füllt.

8. Flüssigkeitströpfchen-Versprühvorrichtung nach einem der vorhergehenden Ansprüche, **dadurch gekennzeichnet, dass** der Zwischenraum aus einem Hauptvolumen (12) und einem Puffervolumen (12a) besteht, wobei das Puffervolumen so dimensioniert ist, dass das Gesamtvolumen des Hauptvolumens (12) zusammen mit dem Puffervolumen (12a) einer maximalen Dosis entspricht, sodass eine gewünschte variable Dosis in Abhängigkeit von dem speziellen Verwendungszweck der Versprühvorrichtung in dem Zwischenraum aufgenommen werden kann, wobei die variable Dosis gleich der maximalen Dosis oder kleiner als diese ist.

9. Verfahren zur Herstellung einer Flüssigkeitströpfchen-Versprühvorrichtung zum Versprühen einer flüssigen Substanz nach Anspruch 6, mit den folgenden Schritten:
a) Abscheiden einer Oxidmaske auf beiden Hauptflächen eines Siliciumträgers, der den ersten Träger (15) bilden soll;
b) Ätzen einer ersten Hauptfläche des ersten Trägers (15), um die dünneren Membranteile (15a) zu erhalten;
c) Aufbringen eines ersten Fotoresists, das große Öffnungen definiert, die dem unteren Teil (20a) der Auslasskanäle (20) entsprechen, auf die andere Hauptfläche des ersten Trägers (15) und anschließend Wegätzen der Oxidmaske, um einen Zugang zu dem Silicium des ersten Trägers (15) freizulegen;
d) Aufbringen eines zweiten Fotoresists, um kleine Öffnungen zu definieren, die dem oberen Teil (20b) der Auslasskanäle (20) entsprechen, und anschließend Verwenden des partiellen Plasma-Ätzens des Siliciums, um einen schmalen Kanal zu erhalten, der schmaler und kürzer ist, als der eigentliche obere Teil (20b) sein wird, und Ablösen des zweiten Fotoresists;
e) Ätzen des freiliegenden Siliciums unter Verwendung des differentiellen Plasma-Ätzens, bis der Träger (15) fast perforiert ist;
f) Verwenden des Plasma-Ätzens auf der anderen Hauptfläche des ersten Trägers (15), um den Träger (15) zu perforieren, wobei das Verhältnis der zu ätzenden Fläche zu der Fläche, die der Düsenöffnung des oberen Teils (20b) entspricht, so ist, dass die Ätzgeschwindigkeit der Düse so gering ist, dass ein Einkerben der Auslassdüse (19) vermieden wird;
g) Ätzen eines zweiten Siliciumträgers, um den zweiten Träger (16) mit einem Zwischenraum (12) darin auszubilden;
h) Verbinden des ersten Trägers (15) mit dem zweiten Träger (16), um das Gehäuse der Flüssigkeitströpfchen-Versprühvorrichtung auszubilden;
i) Bereitstellen von Mitteln zum Zuführen der flüssigen Substanz zu dem Zwischenraum (12) und
j) Bereitstellen des Schwingelements (18).

10. Verfahren zur Herstellung einer Flüssigkeitströpfchen-Versprühvorrichtung zum Versprühen einer flüssigen Substanz nach Anspruch 3, mit den folgenden Schritten:
a) Abscheiden einer Oxidmaske auf beiden Hauptflächen eines Siliciumträgers, der den ersten Träger (15) bilden soll;
b) Ätzen einer ersten Hauptfläche des ersten Trägers (15), um die dünneren Membranteile (15a) zu erhalten;
c) Aufbringen eines ersten Fotoresists, das große Öffnungen definiert, die dem unteren Teil (20a) der Auslasskanäle (20) entsprechen, auf die andere Hauptfläche des ersten Trägers (15) und anschließend Wegätzen der Oxidmaske, um einen Zugang zu dem Silicium des ersten Trägers (15) freizulegen;
d) Aufbringen eines zweiten Fotoresists, um kleine Öffnungen zu definieren, die dem oberen Teil (20b) der Auslasskanäle (20) entsprechen, und anschließend Verwenden des partiellen Plasma-Ätzens des Siliciums, um einen schmalen Kanal zu erhalten, der schmaler und kürzer ist, als der eigentliche obere Teil (20b) sein wird, und Ablösen des zweiten Fotoresists;
e) Ätzen des freiliegenden Siliciums unter Verwendung des differentiellen Plasma-Ätzens, bis der Träger (15) fast perforiert ist;
f) Aufbringen eines dritten Fotoresists, um eine große Öffnung zu definieren, die dem Zwischenraum (12) entspricht, und anschließend Verwenden des Plasma-Ätzens, um die Aussparung zu erhalten, die den Zwischenraum (12) bildet, und um den Träger (15) zu perforieren, wobei das Verhältnis der zu ätzenden Fläche, die dem Zwischenraum entspricht, zu der Fläche, die der Düsenöffnung des oberen Teils (20b) entspricht, so ist, dass die Ätzgeschwindigkeit der Düse so gering ist, dass ein Einkerben der Auslassdüse (19) vermieden wird;
g) Bereitstellen von Mitteln zum Zuführen der flüssigen Substanz zu dem Zwischenraum (12);
h) Bereitstellen des Schwingelements (18) und
i) Verbinden des ersten Trägers (15) mit dem Schwingelement (18), um das Gehäuse der Flüssigkeitströpfchen-Versprühvorrichtung auszubilden.

11. Verfahren zur Herstellung einer Flüssigkeitströpfchen-Versprühvorrichtung zum Versprühen einer flüssigen Substanz nach Anspruch 3, mit den folgenden Schritten:
a) Abscheiden einer Oxidmaske auf beiden Hauptflächen eines Siliciumträgers, der den ersten Träger (15) bilden soll;
b) Ätzen einer ersten Hauptfläche des ersten Trägers (15), um die dünneren Membranteile (15a) zu erhalten;
c) Aufbringen eines ersten Fotoresists, das große Öffnungen definiert, die den unteren Teilen (20a) der Auslasskanäle (20) entsprechen, auf die andere Hauptfläche des ersten Trägers (15) und anschließend Wegätzen der Oxidmaske, um einen Zugang zu dem Silicium des ersten Trägers (15) freizulegen;
d) Aufbringen eines zweiten Fotoresists, um kleine Öffnungen zu definieren, die den oberen Teilen (20b) der Auslasskanäle (20) entsprechen, und anschließend Verwenden des partiellen Plasma-Ätzens des Siliciums, um einen schmalen Kanal zu erhalten, der schmaler und kürzer ist, als der eigentliche obere Teil (20b) sein wird, und Ablösen des zweiten Fotoresists;
e) Ätzen des freiliegenden Siliciums unter Verwendung des differentiellen Plasma-Ätzens, bis der Träger (15) fast perforiert ist;
f) Aufbringen eines dritten Fotoresists, um eine große Öffnung zu definieren, die dem Zwischenraum (12) entspricht, und anschließend Verwenden des Plasma-Ätzens, um die Aussparung zu erhalten, die den Zwischenraum (12) bildet, und um den Träger (15) zu perforieren, wobei das Verhältnis der zu ätzenden Fläche, die dem Zwischenraum entspricht, zu der Fläche, die der Düsenöffnung des oberen Teils (20b) entspricht, so ist, dass die Ätzgeschwindigkeit der Düse so gering ist, dass ein Einkerben der Auslassdüse (19) vermieden wird;
g) Ätzen eines zweiten Siliciumträgers, um den zweiten Siliciumträger (16) auszubilden;
h) Verbinden des ersten Trägers (15) mit dem zweiten Träger (16), um das Gehäuse der Flüssigkeitströpfchen-Versprühvorrichtung auszubilden;
i) Bereitstellen von Mitteln zum Zuführen der flüssigen Substanz zu dem Zwischenraum (12) und
h) Bereitstellen des Schwingelements (18).

12. Verfahren zur Herstellung einer Flüssigkeitströpfchen-Versprühvorrichtung nach Anspruch 9, 10 oder 11, das weiterhin nach dem Schritt e) und vor dem Schritt g) einen Schritt des Aufbringens eines selektiven hydrophilen Überzugs aufweist, um eine Schutzschicht um die Innenfläche des Zwischenraums (12) und/oder der Auslasskanäle (20) herzustellen, um eine Verunreinigung der flüssigen Substanz mit dem Material dieser Flächen zu vermeiden und die Benetzbarkeit in bestimmten Teilen zu verbessern.

13. Verfahren zur Herstellung einer Flüssigkeitströpfchen-Versprühvorrichtung nach Anspruch 12, das weiterhin den folgenden Schritt aufweist: Aufbringen eines selektiven hydrophoben Überzugs in den Bereichen des Zwischenraums (12), die den hydrophilen Überzug nicht haben, und auf der Außenfläche des ersten Trägers (15).

14. Verfahren zur Herstellung einer Flüssigkeitströpfchen-Versprühvorrichtung nach Anspruch 11, das weiterhin den folgenden zusätzlichen Schritt aufweist: Plasma-Ätzen der ersten Hauptfläche des ersten Trägers (15), das im Passivierungsmodus unterbrochen wird, um eine hydrophobe Oberflächengüte zu erhalten, wobei die gleiche Produktionsanlage wie für die anderen differentiellen Plasma-Ätzschritte verwendet wird.

15. Verfahren zur Herstellung einer Flüssigkeitströpfchen-Versprühvorrichtung nach Anspruch 13 oder 14, das weiterhin den Schritt des Aufbringens eines harten amorphen Kohlenstofffilms auf dem hydrophoben Überzug aufweist.

16. Verfahren zur Herstellung einer Flüssigkeitströpfchen-Versprühvorrichtung zum Versprühen einer flüssigen Substanz nach Anspruch 3, mit den folgenden Schritten:
a) Abscheiden einer Oxidmaske auf beiden Hauptflächen eines Siliciumträgers, der den ersten Träger (15) bilden soll;
b) Ätzen einer ersten Hauptfläche des ersten Trägers (15), um die dünneren Membranteile (15a) zu erhalten;
c) Aufbringen eines ersten Fotoresists, das schmale Öffnungen definiert, die dem oberen Teil (20b) der Auslasskanäle (20) entsprechen, auf die andere Hauptfläche des ersten Trägers (15) und anschließend Wegätzen der Oxidmaske, um einen Zugang zu dem Silicium des ersten Trägers (15) freizulegen;
d) Ätzen des Siliciums, um ein Durchgangsloch, das durch den ersten Träger (15) geht, so zu erhalten, dass keine Einkerbung auftritt;
e) Aufbringen einer Schutzschicht (25) auf die inneren Seitenwände des Durchgangslochs;
f) Aufbringen eines zweiten Fotoresists auf die andere Hauptfläche des ersten Trägers (15), um große Öffnungen zu definieren, die dem unteren Teil (20a) der Auslasskanäle (20 entsprechen, und anschließend Ätzen des Siliciums, um einen großen Kanal zu erhalten, der dem unteren Teil (20a) entspricht, und Ablösen des zweiten Fotoresists;
g) Aufbringen einer Schutzschicht (25) auf die inneren Seitenwände des nunmehr abgestuften Durchgangslochs, das dem abgestuften Auslasskanal (20) entspricht;
h) Aufbringen eines dritten Fotoresists auf die andere Hauptfläche des ersten Trägers (15), um eine große Öffnung zu definieren, die dem Zwischenraum (12) entspricht, und anschließend Ätzen des Siliciums, um die abgestufte Aussparung zu erhalten, die den Zwischenraum (12) bildet;
i) Entfernen der Schutzschicht von den inneren Seitenwänden des Durchgangslochs;
j) Bereitstellen von Mitteln zum Zuführen der flüssigen Substanz zu dem Zwischenraum (12);
k) Bereitstellen des Schwingelements (18) und
l) Verbinden des ersten Trägers (15) mit dem Schwingelement (18), um das Gehäuse der Flüssigkeitströpfchen-Versprühvorrichtung auszubilden.

17. Verfahren zur Herstellung eines Düsenkörpers einer Flüssigkeitströpfchen-Versprühvorrichtung, der aus einem Träger mit mindestens einem Membranteil besteht, der dünner als der Rest des Trägers ist, wobei Auslassmittel in dem Membranteil angeordnet sind, die mindestens eine Auslassdüse (19), die in einer ersten Hauptfläche des ersten Trägers (15) angeordnet ist, und mindestens einen abgestuften Auslasskanal (20) aufweisen, der eine Hauptfläche des Trägers mit der anderen Hauptfläche des Trägers verbindet, wobei der Auslasskanal (20) gerade Seitenwände sowie einen breiten unteren Teil (20a) und einen schmalen oberen Teil (20b) hat, mit den folgenden Schritten:
a) Abscheiden einer Oxidmaske auf beiden Flächen des Trägers, der den Düsenkörper bilden soll;
b) Ätzen einer ersten Hauptfläche des Trägers, um die dünneren Membranteile (15a) zu erhalten;
c) Aufbringen eines ersten Fotoresists, das große Öffnungen definiert, die den unteren Teilen (20a) der Auslasskanäle (20) entsprechen, auf die andere Hauptfläche des Trägers und anschließend Wegätzen der Oxidmaske, um einen Zugang zu dem Silicium des Trägers freizulegen;
d) Aufbringen eines zweiten Fotoresists, um kleine Öffnungen zu definieren, die den oberen Teilen (20b) der Auslasskanäle (20) entsprechen, und anschließend Verwenden des partiellen Plasma-Ätzens des Siliciums, um einen schmalen Kanal zu erhalten, der schmaler und kürzer ist, als der eigentliche obere Teil (20b) sein wird, und Ablösen des zweiten Fotoresists;
e) Ätzen des freiliegenden Siliciums unter Verwendung des differentiellen Plasma-Ätzens, bis der Träger fast perforiert ist; und
f) Plasma-Ätzen der anderen Hauptfläche des Trägers, um den Träger (15) zu perforieren, wobei das Verhältnis der zu ätzenden Fläche des Trägers zu der Fläche, die der Düsenöffnung des oberen Teils (20b) entspricht, so ist, dass die Ätzgeschwindigkeit der Düse so gering ist, dass ein Einkerben der Düse vermieden wird.

## Revendications

1. Dispositif de pulvérisation de gouttelettes liquides pour atomiser une substance liquide, comprenant :
- un logement consistant au moins en un premier substrat (15) ayant au moins une section de membrane (15a) qui est plus mince que le reste dudit premier substrat (15),
- un espace (12) à l'intérieur du logement pour contenir la substance liquide,
- un moyen pour amener ladite substance liquide jusqu'audit espace (12),
- un moyen de sortie agencé dans ladite section de membrane (15a) et comprenant au moins une buse de sortie (19) agencée dans une première surface principale dudit premier substrat (15) et au moins un canal de sortie (20) connectant ledit espace (12) à chacune desdites au moins une buse de sortie (19), et
- un élément vibrant (18) disposé de façon à faire vibrer le liquide dans ledit espace (12) afin d'éjecter ladite substance liquide sous la forme d'une pulvérisation à travers lesdites buses de sortie (19),
**caractérisé en ce que** chaque canal de sortie (20) a une forme en marches ayant une partie inférieure (20a) et une partie supérieure (20b), ladite partie inférieure étant agencée de façon adjacente audit espace (12) et étant d'un diamètre plus grand que ladite partie supérieure (20b), chaque partie de canal de sortie (20a, 20b) ayant des parois latérales rectilignes, et en que
ledit reste dudit premier substrat (15) consiste en sections qui sont plus épaisses que ladite section de membrane (15a) et qui sont situées sur le côté de la première surface principale dudit premier substrat (15).

2. Dispositif de pulvérisation de gouttelettes liquides selon la revendication 1, **caractérisé en outre en ce que** ledit reste dudit premier substrat (15) consiste en des sections plus épaisses ayant des parois latérales s'éloignant desdites sections de membrane (15a).

3. Dispositif de pulvérisation de gouttelettes liquides selon la revendication 1 ou 2, **caractérisé en outre en ce que** ledit premier substrat (15) comporte un renfoncement dans son autre surface principale, qui constitue ledit espace (12).

4. Dispositif de pulvérisation de gouttelettes liquides selon la revendication 1, **caractérisé en outre en ce que** ledit élément vibrant (18) est rond et d'une section transversale plus petite que ledit espace (12) et est agencé pour fonctionner à sa fréquence de résonance fondamentale ou à des harmoniques subséquentes de façon à éjecter ladite substance liquide sous la forme d'une pulvérisation à travers lesdites buses de sortie (19).

5. Dispositif de pulvérisation de gouttelettes liquides selon la revendication 3, dans lequel ledit logement comprend en outre un deuxième substrat (16), et dans lequel ledit espace (12) constitué par ledit renfoncement est fermé par ledit deuxième substrat (16).

6. Dispositif de pulvérisation de gouttelettes liquides selon la revendication 1 ou 2, dans lequel ledit logement comprend en outre un deuxième substrat (16), et dans lequel ledit espace (12) est prévu dans ledit deuxième substrat (16).

7. Dispositif de pulvérisation de gouttelettes liquides selon l'une quelconque des revendications précédentes, comprenant en outre une valve passive (12b) agencée en combinaison physique avec ledit espace (12) pour faciliter le fonctionnement homogène dudit dispositif en permettant un remplissage homogène dudit espace (12) avec ladite substance liquide.

8. Dispositif de pulvérisation de gouttelettes liquides selon l'une quelconque des revendications précédentes, dans lequel ledit espace consiste en un volume principal (12) et un volume tampon (12a), ledit volume tampon étant dimensionné de telle sorte que le volume total dudit volume principal (12) avec ledit volume tampon (12a) correspond à une dose maximum, de façon à ce qu'une dose variable désirée en fonction de l'usage spécifique dudit dispositif de pulvérisation puisse être contenue dans ledit espace, ladite dose variable étant égale ou inférieure à ladite dose maximum.

9. Procédé de fabrication d'un dispositif de pulvérisation de gouttelettes liquides pour atomiser une substance liquide selon la revendication 6, le procédé comprenant les étapes suivantes :
a. dépôt d'une épargne d'oxyde sur les deux surfaces principales d'un substrat de silicium qui formera le premier substrat (15),
b. gravure d'une première surface principale dudit premier substrat (15) de façon à obtenir les sections de membrane (15a) plus minces,
c. application d'une première résine photosensible sur l'autre surface principale dudit premier substrat (15) définissant des grandes ouvertures correspondant à la partie inférieure (20a) desdits canaux de sortie (20) et ensuite élimination par gravure de l'épargne d'oxyde pour ouvrir un accès au silicium dudit premier substrat (15),
d. application d'une deuxième résine photosensible pour définir des petites ouvertures correspondant à la partie supérieure (20b) desdits canaux de sortie (20) et ensuite utilisation d'une gravure partielle par plasma du silicium pour obtenir un canal étroit qui est plus étroit et plus court que la partie supérieure (20b) réelle ne le sera, et décapage de la deuxième résine photosensible,
e. gravure du silicium ouvert en utilisant une gravure différentielle par plasma jusqu'à ce que le substrat (15) soit presque percé,
f. utilisation d'une gravure par plasma sur l'autre surface principale dudit premier substrat (15) pour percer ledit substrat (15), le rapport de la surface devant être gravée par rapport à la surface correspondant à l'ouverture de la buse de ladite partie supérieure (20b) étant tel que la vitesse de gravure de ladite buse est suffisamment lente pour éviter un entaillage de la buse de sortie (19),
g. gravure d'un deuxième substrat de silicium de façon à former ledit deuxième substrat (16) avec un espace (12) dans celui-ci,
h. liaison ensemble dudit premier substrat (15) et dudit deuxième substrat (16) pour former ledit logement dudit dispositif de pulvérisation de gouttelettes liquides,
i. prévision d'un moyen pour amener ladite substance liquide jusqu'audit espace (12), et
j. prévision dudit élément vibrant (18).

10. Procédé de fabrication d'un dispositif de pulvérisation de gouttelettes liquides pour atomiser une substance liquide selon la revendication 3, le procédé comprenant les étapes suivantes :
a. dépôt d'une épargne d'oxyde sur les deux surfaces principales d'un substrat de silicium qui formera le premier substrat (15),
b. gravure d'une première surface principale dudit premier substrat (15) de façon à obtenir les sections de membrane (15a) plus minces,
c. application d'une première résine photosensible sur l'autre surface principale dudit premier substrat (15) définissant des grandes ouvertures correspondant à la partie inférieure (20a) desdits canaux de sortie (20) et ensuite élimination par gravure de l'épargne d'oxyde pour ouvrir un accès au silicium dudit premier substrat (15),
d. application d'une deuxième résine photosensible pour définir des petites ouvertures correspondant à la partie supérieure (20b) desdits canaux de sortie (20) et ensuite utilisation d'une gravure partielle par plasma du silicium pour obtenir un canal étroit qui est plus étroit et plus court que la partie supérieure (20b) réelle ne le sera, et décapage de la deuxième résine photosensible,
e. gravure du silicium ouvert en utilisant une gravure différentielle par plasma jusqu'à ce que le substrat (15) soit presque percé,
f. application d'une troisième résine photosensible pour définir une grande ouverture correspondant à l'espace (12) et ensuite utilisation d'une gravure par plasma pour obtenir ledit renfoncement constituant ledit espace (12) et pour percer ledit substrat (15), le rapport de la surface devant être gravée correspondant audit espace par rapport à la surface correspondant à l'ouverture de la buse de ladite partie supérieure (20b) étant tel que la vitesse de gravure de ladite buse est suffisamment lente pour éviter un entaillage de la buse de sortie (19),
g. prévision d'un moyen pour amener ladite substance liquide jusqu'audit espace (12),
h. prévision dudit élément vibrant (18), et
i. liaison ensemble dudit premier substrat (15) et dudit élément vibrant (18) pour former ledit logement dudit dispositif de pulvérisation de gouttelettes liquides.

11. Procédé de fabrication d'un dispositif de pulvérisation de gouttelettes liquides pour atomiser une substance liquide selon la revendication 3, le procédé comprenant les étapes suivantes :
a. dépôt d'une épargne d'oxyde sur les deux surfaces principales d'un substrat de silicium qui formera le premier substrat (15),
b. gravure d'une première surface principale dudit premier substrat (15) de façon à obtenir les sections de membrane (15a) plus minces,
c. application d'une première résine photosensible sur l'autre surface principale dudit premier substrat (15) définissant des grandes ouvertures correspondant aux parties inférieures (20a) desdits canaux de sortie (20) et ensuite élimination par gravure de l'épargne d'oxyde pour ouvrir un accès au silicium dudit premier substrat (15),
d. application d'une deuxième résine photosensible pour définir des petites ouvertures correspondant aux parties supérieures (20b) desdits canaux de sortie (20) et ensuite utilisation d'une gravure partielle par plasma du silicium pour obtenir un canal étroit qui est plus étroit et plus court que la partie supérieure (20b) réelle ne le sera, et décapage de la deuxième résine photosensible,
e. gravure du silicium ouvert en utilisant une gravure différentielle par plasma jusqu'à ce que le substrat (15) soit presque percé,
f. application d'une troisième résine photosensible pour définir une grande ouverture correspondant à l'espace (12) et ensuite utilisation d'une gravure par plasma pour obtenir ledit renfoncement constituant ledit espace (12) et pour percer ledit substrat (15), le rapport de la surface devant être gravée correspondant audit espace par rapport à la surface correspondant à l'ouverture de la buse de ladite partie supérieure (20b) étant tel que la vitesse de gravure de ladite buse est suffisamment lente pour éviter un entaillage de la buse de sortie (19),
g. gravure d'un deuxième substrat de silicium de façon à former ledit deuxième substrat (16),
h. liaison ensemble dudit premier substrat (15) et dudit deuxième substrat (16) pour former ledit logement dudit dispositif de pulvérisation de gouttelettes liquides,
i. prévision d'un moyen pour amener ladite substance liquide jusqu'audit espace (12), et
j. prévision dudit élément vibrant (18).

12. Procédé de fabrication d'un dispositif de pulvérisation de gouttelettes liquides selon la revendication 9, 10 ou 11, comprenant en outre, après l'étape « e » et avant l'étape « g », une étape d'application d'un revêtement hydrophile sélectif pour créer une couche protectrice autour de la surface intérieure de l'espace (12) et/ou des canaux de sortie (20) pour éviter une quelconque contamination de la substance liquide par la matière de ces surfaces et pour améliorer la mouillabilité dans certaines parties.

13. Procédé de fabrication d'un dispositif de pulvérisation de gouttelettes liquides selon la revendication 12, comprenant en outre l'étape d'application d'un revêtement hydrophobe sélectif dans les zones dudit espace (12) qui ne contiennent pas le revêtement hydrophile et sur la surface extérieure dudit premier substrat (15).

14. Procédé de fabrication d'un dispositif de pulvérisation de gouttelettes liquides selon la revendication 11, comprenant en outre une étape additionnelle de gravure par plasma de la première surface principale dudit premier substrat (15) qui est stoppée dans le mode de passivation pour donner une qualité de surface hydrophobe en utilisant le même équipement de production que pour les autres étapes de gravure différentielle par plasma.

15. Procédé de fabrication d'un dispositif de pulvérisation de gouttelettes liquides selon la revendication 13 ou 14, comprenant en outre l'étape d'application d'un film de carbone amorphe dur sur ledit revêtement hydrophobe.

16. Procédé de fabrication d'un dispositif de pulvérisation de gouttelettes liquides pour atomiser une substance liquide selon la revendication 3, le procédé comprenant les étapes suivantes :
a. dépôt d'une épargne d'oxyde sur les deux surfaces principales d'un substrat de silicium qui formera le premier substrat (15),
b. gravure d'une première surface principale dudit premier substrat (15) de façon à obtenir les sections de membrane (15a) plus minces,
c. application d'une première résine photosensible sur l'autre surface principale dudit premier substrat (15) définissant des ouvertures étroites correspondant à la partie supérieure (20b) desdits canaux de sortie (20) et ensuite élimination par gravure de l'épargne d'oxyde pour ouvrir un accès au silicium dudit premier substrat (15),
d. gravure du silicium pour obtenir un trou traversant le premier substrat (15) de façon à ce qu'il n'y ait pas d'entaillage,
e. application d'un revêtement protecteur (25) aux parois latérales intérieures dudit trou traversant,
f. application d'une deuxième résine photosensible sur l'autre surface principale dudit premier substrat (15) pour définir des grandes ouvertures correspondant à la partie inférieure (20a) desdits canaux de sortie (20) et ensuite gravure du silicium pour obtenir un grand canal correspondant à la partie inférieure (20a), et décapage de la deuxième résine photosensible,
g. application d'un revêtement protecteur (25) aux parois latérales intérieures du trou traversant maintenant en forme de marches correspondant au canal de sortie (20) en forme de marches,
h. application d'une troisième résine photosensible sur l'autre surface principale dudit premier substrat (15) pour définir une grande ouverture correspondant à l'espace (12) et ensuite gravure du silicium pour obtenir ledit renfoncement en forme de marches constituant ledit espace (12),
i. élimination dudit revêtement protecteur desdites parois latérales intérieures dudit trou traversant,
j. prévision d'un moyen pour amener ladite substance liquide jusqu'audit espace (12),
k. prévision dudit élément vibrant (18), et
l. liaison ensemble dudit premier substrat (15) et dudit élément vibrant (18) pour former ledit logement dudit dispositif de pulvérisation de gouttelettes liquides.

17. Procédé de fabrication d'un corps de buse d'un dispositif de pulvérisation de gouttelettes liquides, ledit corps de buse consistant en un substrat ayant au moins une section de membrane qui est plus mince que le reste du substrat, dans lequel un moyen de sortie est agencé dans ladite section de membrane qui comprend au moins une buse de sortie (19) agencée dans une première surface principale dudit premier substrat (15) et au moins un canal de sortie (20) en forme de marches connectant une surface principale du substrat à l'autre surface principale du substrat, ledit canal de sortie (20) ayant des parois latérales rectilignes et ayant une partie large inférieure (20a) et une partie étroite supérieure (20b),
le procédé comprenant les étapes suivantes :
a. dépôt d'une épargne d'oxyde sur les deux surfaces dudit substrat qui formera le corps de buse,
b. gravure d'une première surface principale dudit substrat de façon à obtenir les sections de membrane (15a) plus minces,
c. application d'une première résine photosensible sur l'autre surface principale dudit substrat définissant des grandes ouvertures correspondant aux parties inférieures (20a) desdits canaux de sortie (20) et ensuite élimination par gravure de ladite épargne d'oxyde pour ouvrir l'accès au silicium dudit substrat,
d. application d'une deuxième résine photosensible pour définir des petites ouvertures correspondant aux parties supérieures (20b) desdits canaux de sortie (20) et ensuite utilisation d'une gravure partielle par plasma du silicium pour obtenir un canal étroit qui est plus étroit et plus court que la partie supérieure (20b) réelle ne le sera, et décapage de la deuxième résine photosensible,
e. gravure du silicium ouvert en utilisant une gravure différentielle par plasma jusqu'à ce que le substrat soit presque percé,
f. gravure par plasma de l'autre surface principale dudit substrat de façon à percer ledit substrat (15), le rapport de la surface devant être gravée dudit substrat par rapport à la surface correspondant à l'ouverture de la buse de ladite partie supérieure (20b) étant tel que la vitesse de gravure de ladite buse est suffisamment lente pour éviter un entaillage de la buse.
